(19) Europäisches Patentamt
European Patent Office
Office européen des brevets

(11) Publication number: **0 468 292 A2**

# (12) EUROPEAN PATENT APPLICATION

(21) Application number: **91111562.4**

(22) Date of filing: **11.07.91**

(51) Int. Cl.5: **C08G 59/32**, C08G 59/40, C08L 63/00, B32B 27/38

(30) Priority: **23.07.90 JP 193067/90**

(43) Date of publication of application: **29.01.92 Bulletin 92/05**

(84) Designated Contracting States: **DE FR GB IT**

(71) Applicant: **MITSUBISHI RAYON CO., LTD**
**3-19, Kyobashi 2-chome Chuo-ku**
**Tokyo(JP)**

(72) Inventor: **Tada, Hisashi, Mitsubishi Rayon Company Ltd.**
**1-60, Sunadabashi 4-chome, Higashi-ku**
**Nagoya-shi, Aichi(JP)**
Inventor: **Shiraishi, Yoshinobu, Mitsubishi Rayon Co., Ltd.**
**1-60, Sunadabashi 4-chome, Higashi-ku**
**Nagoya-shi, Aichi(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

## (54) Epoxy resin composition for composite material.

(57) An epoxy resin composition for fiber reinforced composite materials comprises:
(A) an epoxy component comprising 50 to 100% by weight of an epoxy resin having three or more epoxy groups,
(B) at least one compound selected from the group consisting of dicyandiamide, 2,6-xylenylbiguanide, o-tolylbiguanide, diphenylguanidine, adipyl dihydrazide, azelayl dihydrazide, and isophthalic acid dihydrazide,
(C) a specific urea compound, and
(D) a reaction product of an amide or amine with a specific epoxy compound.



Rank Xerox (UK) Business Services

## FIELD OF THE INVENTION

The present invention relates to an epoxy resin composition for producing high strength composite materials. The composition is composed of a specific polyglycidyl derivative and an epoxy derivative, and has an extended pot life.

## BACKGROUND OF THE INVENTION

Since fiber reinforced composite materials containing carbon fibers, alumina fibers or all aromatic polyamide fibers as a reinforcing material have excellent mechanical performance, they are used for structural parts in various industries and also for sporting and leisure goods, or the like. Although various materials have been used as a matrix resin for carbon fiber composite materials, epoxy resins have been widely used because, for example, they are excellent in mechanical properties, have no volatile components at the time of curing, shrink less upon curing, and are excellent in adhesion to carbon fibers (Japanese Patent Publication No. 40975/1983, and Japanese Patent Application Laid-Open No. 57416/1987). The 0° direction bending strength (converted as the value of that having a fiber volume content of 60%) of the composite materials in which reinforcing fibers are laminated in one-direction has been on the order of 190 $kg/mm^2$ and their interlayer shear strength has been on the order of 10 $kg/mm^2$. Considering these conditions, various studies to improve the mechanical or physical properties and to improve the heat resistance of such composite materials have been made. In Japanese Patent Publication No. 15570/1987, as a composition that can provide a composite material high in interlayer shear strength, an epoxy resin composition comprising diaminodiphenylsulfone and an epoxy resin consisting of N,N-diglycidyl-aminophenylglycidyl-ether and/or N,N-diglycidylaniline is disclosed. When this composition was used, a composite material having an interlayer shear strength of 12.8 $kg/mm^2$ could be obtained, but the highest 0° direction bending strength was 192 $kg/mm^2$. Further, since the curing temperature at which the composite material is molded is as high as 170°C, and it is required to carry out the postcuring at a temperature that is as high as 190°C, the epoxy resin composition lacked general-purpose properties. Japanese Patent Application Laid-Open Nos. 183340/1987, 183341/1987, and 183342/1987 disclose, as resin compositions that can improve physical properties and heat resistance of the composite materials, three compositions, i.e., a composition (I) consisting of a polyglycidyl derivative of 4-amino-m-cresol and/or 4-amino-o-cresol and diaminodiphenylsulfone and/or diaminodiphenylmethane, a composition (II) consisting of N,N,N',N'-tetraglycidyl(amino-phenyl)methane and/or its condensate, a polyglycidyl derivative of 4-amino-m-cresol and/or 4-amino-o-cresol and/or its condensate, and diaminodiphenylsulfone and/or diaminodiphenylmethane, and a composition (III) consisting of a phenol-novolak type epoxy resin, a cresol-novolak type epoxy resin and/or a bisphenol A diglycidyl ether type epoxy resin, a polyglycidyl derivative of 4-amino-m-cresol and/or 4-amino-o-cresol and/or its condensate, and dicyandiamide or an acid hydrazide type compound. Although a 0° direction bending strength of 215 $kg/mm^2$ and an interlayer shear strength of 13.6 $kg/mm^2$ can be accomplished by the compositions (I) and (II), the curing requires 1 hour at 150°C, and the postcuring requires 4 hours at 180°C, which is a long period at a high temperature. In the case of the composition (III) whose curing requires 1 hour at 120°C and whose postcuring requires 2 hours at 130°C, the 0° direction bending strength is 210 $kg/mm^2$, and the interlayer shear strength is 11.2 $kg/mm^2$, the values being rather small.

Also, an epoxy resin composition has been developed as a matrix resin for composite materials which can be cured at a temperature of 150°C or lower and the 0° direction bending strength and interlayer shear strength of the composite materials from the epoxy resin composition are higher than 220 $kg/mm^2$ and higher than 10 $kg/mm^2$, respectively (Japanese Patent Application Laid-Open No. 287130/1989). The prepregs prepared from this epoxy resin composition were stable for 15 days at a temperature of 25°C and thus the prepregs were satisfactory in practical use. However, the development of an epoxy resin composition from which prepregs having a longer pot life can be prepared has been desired from the viewpoint of transportation and storage of the prepregs.

As a result of the research and development of epoxy resin compositions in consideration of the situation explained in the foregoing, it has now been found that an epoxy resin composition, for composite materials, from which prepregs having an extended pot life and composite materials having excellent mechanical performance are prepared can be obtained by using a specific epoxy compound, curing agent, curing accelerator and specific product as essential components of the composition.

## SUMMARY OF THE INVENTION

An object of the present invention is to provide an epoxy resin composition from which composite materials having a high 0° direction bending strength and a high interlayer shear strength can be prepared.

Another object of the present invention is to provide an epoxy resin composition from which prepregs having a long pot life can be prepared.

Further objects will be apparent to those skilled in the art from the following detailed description and appended claims.

The foregoing objects of the present invention are achieved by an epoxy resin composition for composite materials comprising:

(A) an epoxy component comprising 50 to 100% by weight of an epoxy resin selected from the group consisting of an epoxy resin (A-1) represented by the following general formula:

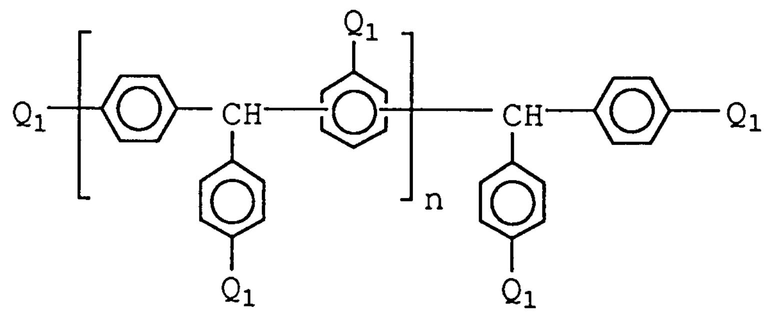 ··· (1)

wherein $Q_1$ represents

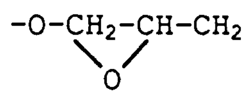

group, and n is a numeral of 0 to 5, and an epoxy resin (A-2) represented by the following general formula:

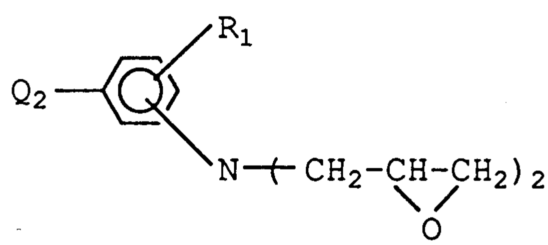 ··· (2)

wherein $Q_2$ represents the same group as $Q_1$ in the general formula (1), and $R_1$ represents hydrogen atom or methyl group, the epoxy resins (A-1) and (A-2) being present at a ratio of (A-1)/(A-2) = 100/0 to 50/50 by weight,

(B) at least one compound selected from the group consisting of dicyandiamide, 2,6-xylenylbiguanide, o-tolylbiguanide, diphenylguanidine, adipyl dihydrazide, azelayl dihydrazide, and isophthalic acid dihydrazide,

(C) a compound represented by the following general formula:

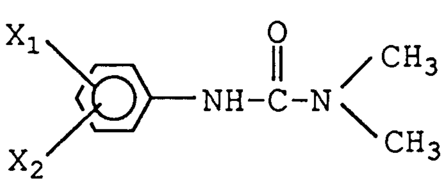 ··· (3)

wherein $X_1$ and $X_2$, which may be the same or different, each represents -H, -Cℓ, -Br, $-NO_2$, $-CH_3$, $-OCH_3$, $-OC_2H_5$, or

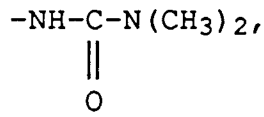

and

(D) a reaction product obtained by reacting an amide or amine represented by the general formula:

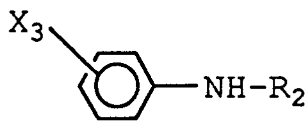

... (4)

wherein $X_3$ represents -H, -Cℓ, -Br or -OH, and $R_2$ represents -H, $-COCH_3$, or $-CO-CH_2-CO-CH_3$ with an epoxy compound represented by the general formula:

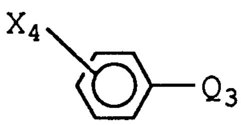

... (5)

or

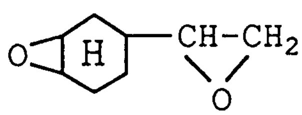

... (6)

wherein $X_4$ is the same as $X_3$ in the general formula (4) and $Q_3$ represent the same group as $Q_1$ in the general formula (1).

DESCRIPTION OF THE INVENTION

Both the epoxy resin (A-1) represented by the general formula (1) and the epoxy resin (A-2) represented by the general formula (2) have more epoxy groups than the bisphenol A type epoxy resins conventionally used. Thus, when the epoxy resins (A-1) and (A-2) are used as an epoxy component of the composition, the density of crosslinking in the resin composition and the elasticity of the resin composition after curing will be increased, leading to a high performance composite material. The first characteristic of the present invention resides in the use of such polyfunctional epoxy resins.

Specific examples of the epoxy resin (A-1) includes Epicoat 1032 (hereinafter referred as Ep. 1032) and Ep. YL 933 (both manufactured by Yuka Shell Epoxy Co., Ltd.), and EPPN-501H and EPPN-502H (both manufactured by Nippon Kayaku Co., Ltd.).

Preferred epoxy resin (A-2) includes the following compounds:

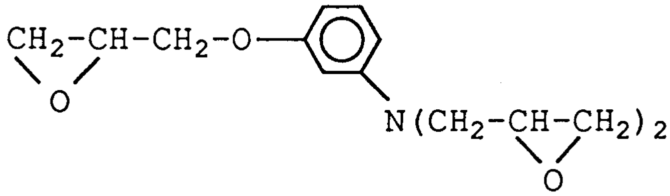 ... (7)

(ELM-120 manufactured by Sumitomo Chemical Co., Ltd.)

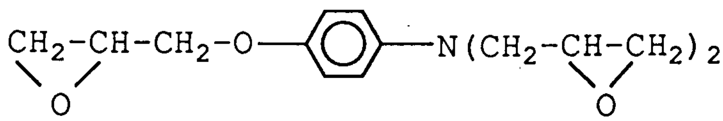 ... (8)

(0500 manufactured by Ciba-Geigy Co., Ltd.)

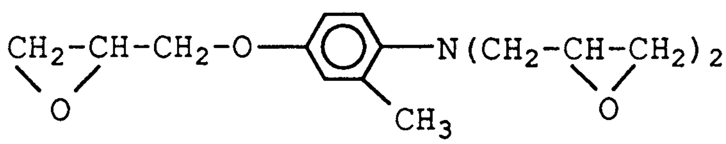 ... (9)

(ELM-100 manufactured by Sumitomo Chemical Co., Ltd.)

Among the epoxy resins represented by the formulas (7) through (9), the compound represented by the formula (9) is most desirable.

In the present invention, the blend ratio of the epoxy resin (A-1) to the epoxy resin (A-2) is (A-1)/(A-2) = 100/0 to 50/50 by weight. When the amount of the epoxy resin (A-2) exceeds 50% by weight, handling of the obtained prepregs becomes difficult.

In the present invention, the epoxy component (A) contains 50 to 100% by weight of epoxy resin (A-1) or a mixture of epoxy resin (A-1) and epoxy resin (A-2). The epoxy component (A) may further contain less than 50% by weight of another type of epoxy resin. Suitable epoxy resins include: glycidyl ethers of bisphenol compounds, such as bisphenol S, bisphenol A and bisphenol F; glycidyl ethers of phenol-or cresol-novolac resins; and tetraglycidylamine of diaminodiphenylmethane or diaminodiphenylsulfone. These compound may be used alone or in combination.

The compound (B) used in the present invention as a curing agent includes dicyandiamide, 2,6-xylenebiguanide, o-tolylbiguanide, diphenylguanidine, adipyl dihydrazide, azelayl dihydrazide, and isophthalic acid dihydrazide, with dicyandiamide being preferable.

The compound (C) used in the present invention as a curing accelerator is represented by the general formula (3), with 3-(3,4-dichlorophenyl)-1,1-dimethylurea being particularly preferable.

The compounds (B) and (C) are used in combination.

By changing the amount of the curing agent (B) and the curing accelerator (C) to be included in the composition, resins having different performances can be obtained.

For instance, by changing the amount of the curing agent (B), the density of crosslinking in the obtained resin will be changed, and thus the elasticity of the resin and the performance of the composite materials can be altered. Taking such facts into consideration, the amount of the curing agent (B) is generally 1 to 10 parts by weight, most preferably 2 to 5 parts by weight per 100 parts by weight of the epoxy component (A).

Since the amount of the curing accelerator (C) will control the curing temperature to be selected, careful consideration will be invited for the amount of the accelerator (C). The amount of the accelerator (C) is generally 1.5 to 15 parts by weight, most preferably 3 to 8 parts by weight per 100 parts by weight of the epoxy component (A).

The reaction product (D) is used in the present invention to improve such characteristics as elastic modulus of the resin composition and further to improve the bending strength and interlayer shear strength of carbon fiber composite materials prepared by using the epoxy resin composition of the present invention

as a matrix resin.

When the reaction product (D) is included in the epoxy resin composition, the product (D) will fill up the space formed between the crosslinks to decrease the water absorption of the matrix resin and thus the decrease of the physical properties of the resins by moisture can be remarkably reduced.

The reaction product (D) is obtained by reacting the amine or amide represented by the general formula (4) with the epoxy compound represented by the general formula (5), the epoxy compound represented by the general formula (6), or a mixture of the epoxy compounds.

Specific examples of the reaction product include a reaction product of p-hydroxyacetanilide or acetanilide with phenyl glycidyl ether and a reaction product of aniline with 4-vinylcyclohexene dioxide. These reaction products are prepared by reacting the compound (4) with the compound (5) or (6) at a molar ratio of 1:0.9 to 1:1.1 at a temperature of 100 to 200 °C without difficulty. Although the reaction products thus obtained may be used as they are, it is preferable to heat the products at a temperature of 50 to 200 °C under reduced pressure of 1 mmHg to separate unreacted components from the products.

The amount of the reaction product (D) to be added is preferably in the range of 5 to 100 parts by weight, more preferably 10 to 50 parts by weight per the 100 parts by weight of the total amount of the epoxy component (A), the compound (B), and the compound (C). When the amount of the reaction product (D) is less than 5 parts by weight or more than 100 parts by weight, mechanical properties of the composite materials tend to become lower.

The resin composition of the present invention is used effectively as an intermediate material for carbon fiber composite materials. The carbon fibers used may be in any form, for example, in the form of a tape, a sheet, a mat, or a fabric wherein carbon fibers are arranged in one direction. Instead of carbon fibers, glass fibers, organic fibers, or metal fibers may be used, which may, of course, also be used together with carbon fibers.

The carbon fiber reinforced composite materials produced by using the epoxy resin compositions of the present invention as a matrix resin have a 0 ° direction bending strength of higher than 220 $kg/mm^2$ and an interlayer shear strength of higher than 10 $kg/mm^2$. Such composite materials can be produced at a curing temperature of lower than 150 °C. Besides, the prepregs from the epoxy resin compositions are excellent in their stability at ambient temperature. Therefore, the epoxy resin compositions of the present invention may expect, as matrix resins, a wide range of applications from the field of sports and leisure, such as fishing rods and golf shafts, to the field of industry, such as automobiles, aircrafts and rockets.

## EXAMPLES

### Reference Example 1

Synthesis of N-[4-(2-hydroxy-3-phenoxypropoxy)-phenyl]-acetamide (D-1)

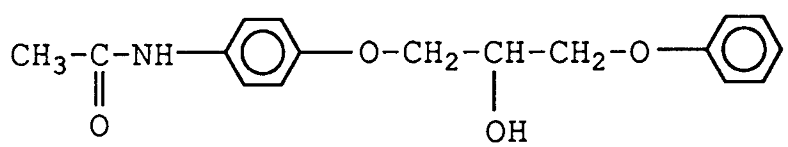

163 g (1 mole) of p-hydroxyacetanilide and 141 g (1.05 mole) of phenylglycidyl ether were mixed and heated at 160 °C for 60 min to obtain a reaction product, the product being a viscous liquid at ambient temperature. The reaction product was heated to 150 °C under a reduced pressure of 1 mmHg to separate unreacted phenylglycidyl ether from the product to give a reaction product (D-1). The infrared absorption spectrum of the reaction product (D-1) was measured and it was confirmed that it contained no epoxy groups. A chloroform solution of the reaction product (D-1) was subjected to gel permeation chromatography, and it was found out that the reaction product (D-1) was a mixture containing, in addition to the 1:1 reaction product, a polymeric compound that resulted from further reactions.

### Reference Example 2

Synthesis of N-phenyl-N-(2-hydroxy-3-phenoxypropyl)-acetacetamide (D-2)

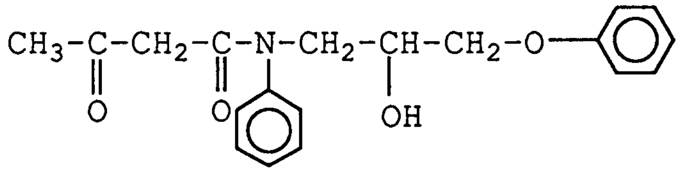

177 g (1 mole) of acetacetanilide and 161 g (1.2 mole) of phenylglycidyl ether were mixed and heated at 170 °C for 1 hr to obtain a reaction product. The reaction product was heated to 150 °C under a reduced pressure of 1 mmHg to separate unreacted phenylglycidyl ether from the product to give a viscous reaction product (D-2). The reaction product (D-2) was subjected to identification in the same manner as in Reference Example 1 to find out that the reaction product (D-2) was a mixture containing, in addition to the 1:1 reaction product, a polymeric compound that resulted from further reactions.

Reference Example 3

Synthesis of N-(2-hydroxy-2-cyclohexeneoxideethyl)-aniline (D-3)

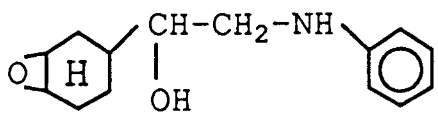

93 g (1 mole) of aniline and 294 g (2.1 mole) of vinylcyclohexene dioxide were mixed and heated at 100 °C for 45 min to obtain a reaction product in viscous liquid state. The reaction product was heated under a reduced pressure in the same manner as in Reference Example 1 to obtain a reaction product (D-3), the product being solid at ambient temperature. The reaction product (D-3) had a melting point of about 110 °C, and was subjected to identification in the same manner as in Reference Example 1 to find out that the reaction product (D-3) was a mixture containing, in addition to the 1:1 reaction product, a polymeric compound that resulted from further reactions.

Example 1

80 parts by weight of an epoxy resin, Ep. 1032S50 (that is an epoxy resin represented by the general formula (1) and manufactured by Yuka Shell Epoxy Co., Ltd.) and 20 parts by weight of an epoxy resin, ELM-100 (represented by the general formula (9) and manufactured by Sumitomo Chemical Co., Ltd.) were thoroughly blended in a kneader. Then, 3 parts by weight of dicyandiamide (DICY), 5 parts by weight of 3-(3,4-dichlorophenyl)-1,1-dimethylurea (DCMU), and 32 parts by weight of the reaction product (D-1) were further added to the kneader and the all components added were thoroughly blended to obtain an epoxy resin composition for prepregs.

The epoxy resin composition was heated about 65 °C and was formed into a thin film on a release paper thereby producing a so-called hot melt film. The hot melt film was wound on a drum with the resin composition facing outside (in other words, with the release paper contacting on the surface of the drum), and carbon fibers (Pyrofil® TR-40 manufactured by Mitsubishi Rayon Co., Ltd.) arranged in one direction were wound on the resin composition. The fibers and the hot melt film were heated at 130 °C, for about 20 sec, so that the resin composition becomes a viscous liquid and impregnates the fibers, and then allowed to cool to form a prepreg. The prepreg thus obtained had an appropriate stickiness and stiffness for further handling which changed little even upon storage for a month at 25 °C. This indicates that the prepreg obtained in this Example had a good storage stability.

The stickiness and stiffness were evaluated by winding a part of the prepreg on a pencil having a diameter of about 8 mm at 25 °C and observing whether breakage or unwinding of the prepreg occurs.

Then, the prepreg was unwound, without storage test, cut into 20 mm lengths, unidirectionally laminated and adjusted so that the content of the carbon fibers after the molding is 60 vol. %. Then the laminated prepreg was placed in an autoclave and was heated at 140°C for 1 hr under a pressure of 6 $kg/cm^2$ to form a carbon fiber reinforced composite material (CFRP).

The CFRP had a 0° direction bending strength of 221 $kg/mm^2$ and an interlayer shear strength of 13.5 $kg/mm^2$.

The methods of measuring the interlayer shear strength and bending strength of the carbon fiber composite material were as follows:

(1) The interlayer shear strength (ILSS)

The interlayer shear strength was measured according to ASTM D-2344. A platelike test piece having a length of 15 mm, a width of 10 mm and a thickness of 2 mm was used. The test piece was placed on fulcrum points (whose tips had a radius of 3.2 mm) with the span interval between them being 8 mm, and an indenter having a tip with a radius of 3.2 mm was pressed on the center of the test piece to carry out a three-point test wherein the cross head speed was 2 mm/min. The interlayer shear strength was calculated according to the formula given below. The span interval was designated L (mm), the thickness of the sample was designated T (mm), the width of the sample was designated W (mm), and the breaking load was designated P (kg).

Interlayer shear strength = 3P/4WT ($kg/mm^2$)

(2) The bending strength (FS//)

The bending strength was measured according to ASTM D-790. A platelike test piece having a length of 100 mm, a width of 10 mm and a thickness of 2 mm was used. The test was carried out in the same manner as the interlayer shear strength test, except that the span interval was 80 mm. The 0° direction bending strength was calculated according to the following formula:

Bending strength = $3PL/2WT^2$ ($kg/mm^2$)

Example 2

Example 1 was repeated in the same way except that the epoxy resins, compounds (B) and (C), and reaction products (D) shown in Table 1 were used, and a different carbon fiber (Pyrofil® TR-30 manufactured by Mitsubishi Rayon Co., Ltd.) was used.

The prepreg thus obtained had an appropriate stickiness and stiffness for further handling which changed little even upon storage for a month at 25°C. This indicates that the prepreg obtained in this Example had a good storage stability.

The abbreviations in Table 1 stand for the epoxy resins or compounds, as follows:

| | |
|---|---|
| ELM-120: | Derivative of m-aminophenyltriglycidyl ether (manufactured by Sumitomo Chemical Co., Ltd.) |
| Ep. 828: | Bisphenol A type epoxy resin (manufactured by Yuka Shell Epoxy Co., Ltd.) |
| Ep. 1001: | Bisphenol A type epoxy resin (manufactured by Yuka Shell Epoxy Co., Ltd.) |
| LCB-100: | Reaction product of Ep. 828 with diaminodiphenyl sulfone (DDS) |
| B-1 : | Diphenylguanidine |
| B-2 : | o-tolylbiguanide |
| B-3 : | Isophthalic acid dihydrazide |
| C-1 : | Compound represented by the general formula (3) wherein $X_1$ is H, $X_2$ is $-NHCON(CH_3)_2$ group. |

The interlayer shear strength and bending strength of the carbon fiber composite materials obtained in the Example are shown in Table 1. From this Table, it can be understood that the composite materials of the present invention provide high performance.

Table 1

| Experiment No. | Epoxy resin (wt%) Ep. 1032 | ELM-100 | ELM-120 | Ep. 828 | Ep. 1001 | LCB-100 | Compound (B) (phr) | Compound (C) (phr) | Reaction product (D) (wt%) | ILSS ($kg/mm^2$) | FS// ($kg/mm^2$) |
|---|---|---|---|---|---|---|---|---|---|---|---|
| 1 | 80 | | 20 | | | | DICY 3 | $X_1=X_2=Cl$ 5 | D-1 30 | 12.8 | 221 |
| 2 | 60 | 20 | | 20 | | | 〃 3 | 〃 5 | 〃 30 | 12.6 | 223 |
| 3 | 60 | 20 | | | 20 | | 〃 3 | 〃 5 | 〃 30 | 12.8 | 222 |
| 4 | 60 | 20 | | | | 20 | 〃 3 | 〃 5 | 〃 30 | 12.2 | 228 |
| 5 | 40 | 20 | | | | | B-1 5 | 〃 4 | 〃 30 | 13.1 | 231 |
| 6 | 80 | 20 | | | | | B-2 2 | 〃 4 | 〃 30 | 13.0 | 230 |
| 7 | 80 | 20 | | | | | B-3 4 | 〃 4 | 〃 30 | 12.5 | 225 |
| 8 | 80 | 20 | | | | | DICY 3 | $X_1=X_2=NO_2$ 4 | 〃 30 | 12.8 | 229 |
| 9 | 80 | 20 | | | | | 〃 3 | C-1 2 | 〃 30 | 13.1 | 230 |
| 10 | 80 | 20 | | | | | 〃 3 | $X_1=X_2=Cl$ 5 | 〃 20 | 12.3 | 225 |
| 11 | 80 | 20 | | | | | 〃 3 | 〃 5 | 〃 50 | 12.4 | 228 |
| 12 | 80 | 20 | | | | | 〃 3 | 〃 5 | D-2 20 | 12.3 | 226 |
| 13 | 80 | 20 | | | | | 〃 3 | 〃 5 | 〃 30 | 13.1 | 230 |
| 14 | 80 | 20 | | | | | 〃 3 | 〃 5 | D-3 10 | 12.2 | 222 |
| 15 | 80 | 20 | | | | | 〃 3 | 〃 5 | 〃 20 | 12.3 | 224 |
| 16 | 100 | | | | | | 〃 3 | 〃 5 | D-1 30 | 13.4 | 223 |
| 17 | 70 | 30 | | | | | 〃 3 | 〃 5 | 〃 30 | 13.0 | 228 |
| 18 | 50 | 50 | | | | | 〃 3 | 〃 5 | 〃 30 | 13.1 | 230 |

Curing: 140°C × 1 hr.    ILSS : Interlayer shear strength    FS// : Bending strength

Comparative Example 1

Example 1 was repeated in the same manner except that the epoxy resins, compounds (B) and (C), and reaction product (D) shown in Table 2 were used. Epoxy resin compositions used in this Comparative

Example are all outside the claimed invention.

The prepreg thus obtained had an appropriate stickiness and stiffness for further handling which changed little even upon storage for a month at 25°C. This indicates that the prepreg obtained in this Comparative Example had a good storage stability.

However, as will be understood from Table 2, the carbon fiber composite meterials thus obtained had considerably lower interlayer shear strengths and bending strengths.

Table 2

| Experiment No. | Epoxy resin (wt%) Ep. 1032 | ELM-100 | ELM-120 | Ep. 828 | Ep. 1001 | LCB-100 | Compound (B) (phr) | Compound (C) (phr) | Reaction product (D) (wt%) | ILSS ($kg/mm^2$) | FS// ($kg/mm^2$) |
|---|---|---|---|---|---|---|---|---|---|---|---|
| 1 | | | | 100 | | | DICY 4 | $X_1$=$X_2$=Cl 5 | D-1 30 | 10.2 | 179 |
| 2 | | | | | 100 | | ″ | ″ | ″ | 10.5 | 181 |
| 3 | | | | | | 100 | ″ | ″ | ″ | 11.5 | 185 |
| 4 | 10 | | | | | 90 | ″ | ″ | ″ | 12.1 | 187 |
| 5 | 40 | | | | | 60 | ″ | ″ | ″ | 11.9 | 190 |
| 6 | 20 | 20 | | | | 60 | ″ | ″ | ″ | 12.0 | 192 |
| 7 | | 40 | | | | 60 | ″ | ″ | ″ | 11.8 | 193 |
| 8 | 80 | 20 | | | | | ″ | ″ | - | 12.5 | 198 |
| 9 | 80 | 20 | | | | | ″ | ″ | 150 | 10.6 | 189 |

Curing: 140°C x 1 hr. ILSS : Interlayer shear strength FS// : Bending strength

Experiment 3

Carbon fiber reinforced composite materials (CFRP) prepared in Example 1, Experiments No. 1 to No. 3 of Example 2, and Experiments No. 1 to No. 3 of Comparative Example 1 were immersed in hot water

(50 °C) for 24 hrs, and the interlayer shear strength and bending strength of the composite materials before and after the immersion were measured. Results thus obtained are shown in Table 3.

From Table 3, it is understood that the carbon fiber reinforced composite materials of the present invention have a low water absorption and thus are low in performance deterioration due to moisture.

Table 3

| Experiment No. | CFRP | Performance of CFRP | | | | |
|---|---|---|---|---|---|---|
| | | Before immersion | | After immersion | | |
| | | ILSS ($kg/mm^2$) | FS// ($kg/mm^2$) | Water absorption (wt%) | ILSS ($kg/mm^2$) | FS// ($kg/mm^2$) |
| 1 | Example 1 | 13.5 | 221 | 0.20 | 12.9 | 215 |
| 2 | Example 2 No.1 | 12.8 | 221 | 0.16 | 12.1 | 218 |
| 3 | " No.2 | 12.6 | 223 | 0.18 | 12.0 | 220 |
| 4 | " No.3 | 12.8 | 222 | 0.15 | 12.2 | 219 |
| 5 | Comp. Ex. No.1 | 10.2 | 179 | 0.89 | 9.5 | 169 |
| 6 | " No.2 | 10.5 | 181 | 0.90 | 9.7 | 173 |
| 7 | " No.3 | 11.5 | 185 | 0.95 | 10.8 | 175 |

## Claims

**1.** An epoxy resin composition for composite materials comprising:

(A) an epoxy component comprising 50 to 100% by weight of an epoxy resin selected from the group consisting of an epoxy resin (A-1) represented by the following general formula:

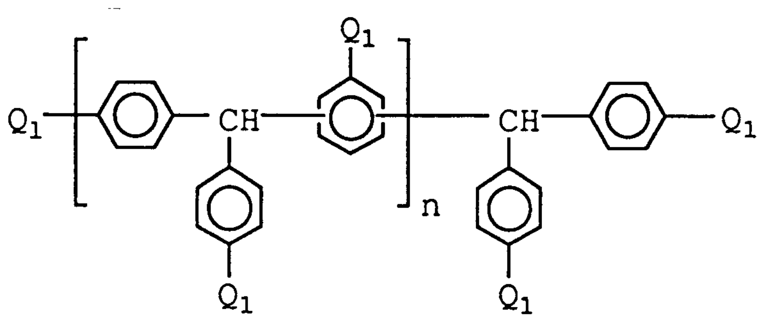

··· (1)

wherein $Q_1$ represents

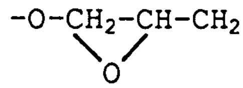

group, and n is a numeral of 0 to 5, and an epoxy resin (A-2) represented by the following general formula:

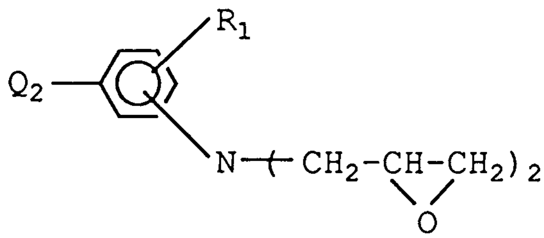

... (2)

wherein $Q_2$ represents the same group as $Q_1$ in the general formula (1), and $R_1$ represents hydrogen atom or methyl group, the epoxy resins (A-1) and (A-2) being present at a ratio of (A-1)/(A-2) = 100/0 to 50/50 by weight,

(B) at least one compound selected from the group consisting of dicyandiamide, 2,6-xylenylbiguanide, o-tolylbiguanide, diphenylguanidine, adipyl dihydrazide, azelayl dihydrazide, and isophthalic acid dihydrazide,

(C) a compound represented by the following general formula:

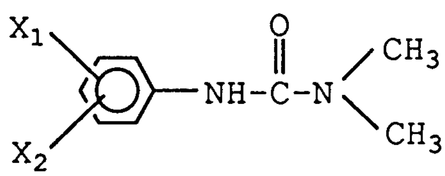

... (3)

wherein $X_1$ and $X_2$, which may be the same or different, each represents -H, -Cℓ, -Br, $-NO_2$, $-CH_3$, $-OCH_3$, $-OC_2H_5$, or

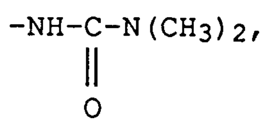

and

(D) a reaction product obtained by reacting an amide or amine represented by the general formula:

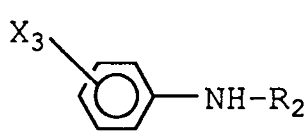

... (4)

wherein $X_3$ represents -H, -Cℓ, -Br or -OH, and $R_2$ represents -H, $-COCH_3$, or $-CO-CH_2-CO-CH_3$with an epoxy compound represented by the general formula:

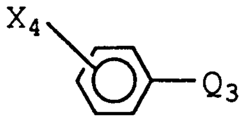

... (5)

or

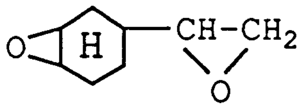

... (6)

wherein $X_4$ is the same as $X_3$ in the general formula (4) and $Q_3$ represent the same group as $Q_1$ in the general formula (1).

2. The composition of claim 1, wherein the amount of the compound (B) is 1 to 10 parts by weight per 100 parts by weight of the epoxy component (A).

3. The composition of claim 1, wherein the compound (B) is dicyandiamide.

4. The composition of claim 1, wherein the amount of the compound (C) is 1.5 to 15 parts by weight per 100 parts by weight of the epoxy component (A).

5. The composition of claim 1, wherein the compound (C) is 3-(3,4-dichlorophenyl)-1,1-dimethylurea.

6. The composition of claim 1, wherein the amount of the reaction product (D) is 5 to 100 parts by weight per 100 parts by weight of the total amount of the epoxy component (A), the compound (B), and the compound (C).

7. The composition of claim 1, wherein the reaction product (D) is a reaction product of p-hydroxyacetanilide or acetanilide with phenyl glycidyl ether.

8. The composition of claim 1, wherein the reaction product (D) is a reaction product of aniline with 4-vinyl cyclohexene dioxide.

9. The composition of claim 1, wherein the resin composition further contains up to 50% by weight of an epoxy resin other than the epoxy resins (A-1) and (A-2).

10. A prepreg composition for composite materials comprising:
(I) an epoxy resin composition comprising:
(A) an epoxy component comprising 50 to 100% by weight of an epoxy resin selected from the group consisting of an epoxy resin (A-1) represented by the following general formula:

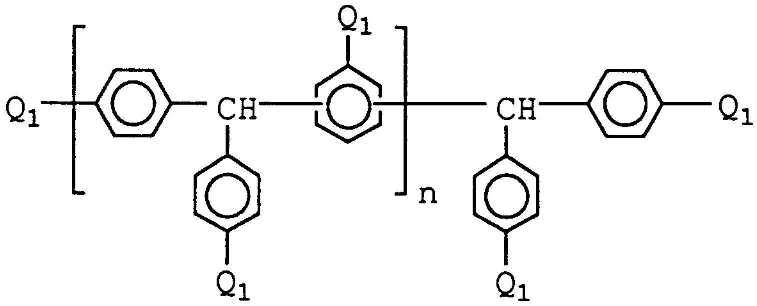

... (1)

wherein $Q_1$ represents

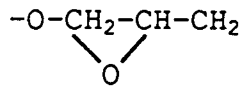

group, and n is a numeral of 0 to 5, and an epoxy resin (A-2) represented by the following general formula:

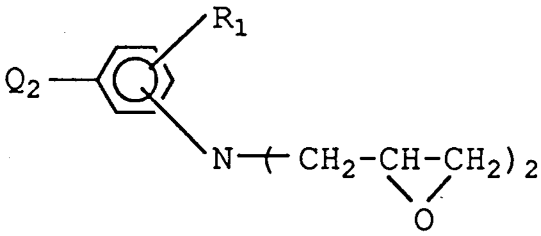

... (2)

wherein $Q_2$ represents the same group as $Q_1$ in the general formula (1), and $R_1$ represents hydrogen atom or methyl group, the epoxy resins (A-1) and (A-2) being present at a ratio of (A-1)-/(A-2) = 100/0 to 50/50 by weight,

(B) at least one compound selected from the group consisting of dicyandiamide, 2,6-xylenylbiguanide, o-tolylbiguanide, diphenylguanidine, adipyl dihydrazide, azelayl dihydrazide, and isophthalic acid dihydrazide,

(C) a compound represented by the following general formula:

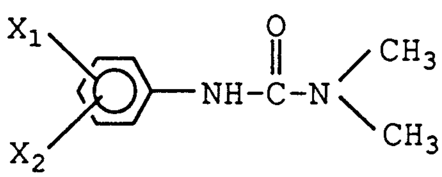

... (3)

wherein $X_1$ and $X_2$, which may be the same or different, each represents -H, -Cℓ, -Br, $-NO_2$, $-CH_3$, $-OCH_3$, $-OC_2H_5$, or

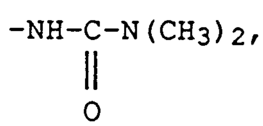

and

(D) a reaction product obtained by reacting an amide or amine represented by the general formula:

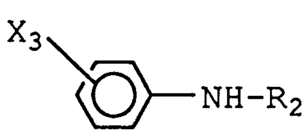

... (4)

wherein $X_3$ represents -H, -Cℓ, -Br or -OH, and $R_2$ represents -H, $-COCH_3$, or $-CO-CH_2-CO-CH_3$

with an epoxy compound represented by the general formula:

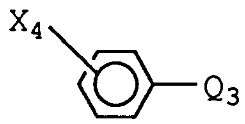

··· (5)

or

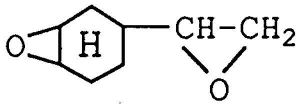

··· (6)

wherein $X_4$ is the same as $X_3$ in the general formula (4) and $Q_3$ represent the same group as $Q_1$ in the general formula (1); and

(II) a unidirectionally oriented fiber reinforcement.

**11.** The composition of claim 10, wherein the amount of the compound (B) is 1 to 10 parts by weight per 100 parts by weight of the epoxy component (A).

**12.** The composition of claim 10, wherein the compound (B) is dicyandiamide.

**13.** The composition of claim 10, wherein the amount of the compound (C) is 1.5 to 15 parts by weight per 100 parts by weight of the epoxy component (A).

**14.** The composition of claim 10, wherein the compound (C) is 3-(3,4-dichlorophenyl)-1,1-dimethylurea.

**15.** The composition of claim 10, wherein the amount of the reaction product (D) is 5 to 100 parts by weight per 100 parts by weight of the total amount of the epoxy component (A), the compound (B), and the compound (C).

**16.** The composition of claim 10, wherein the reaction product (D) is a reaction product of p-hydroxyacetanilide or acetanilide with phenyl glycidyl ether.

**17.** The composition of claim 10, wherein the reaction product (D) is a reaction product of aniline with 4-vinyl cyclohexene dioxide.

**18.** The composition of claim 10, wherein the resin composition further contains up to 50% by weight of an epoxy resin other than the epoxy resins (A-1) and (A-2).

**19.** The composition of claim 10, wherein the fiber reinforcement comprises glass fibers, organic fibers or metal fibers.

**20.** The composition of claim 10, wherein the fiber reinforcement comprises carbon fibers.

**21.** A cured carbon fiber reinforced composite material comprising:

(I) a cured epoxy resin composition comprising:

(A) an epoxy component comprising 50 to 100% by weight of an epoxy resin selected from the group consisting of an epoxy resin (A-1) represented by the following general formula:

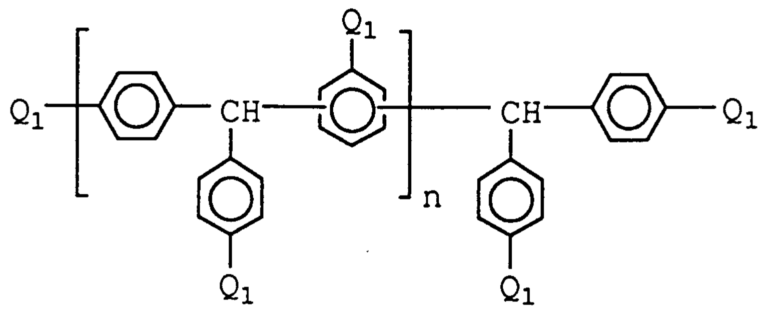 ··· (1)

wherein $Q_1$ represents

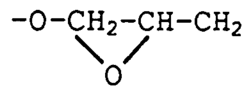

group, and n is a numeral of 0 to 5, and an epoxy resin (A-2) represented by the following general formula:

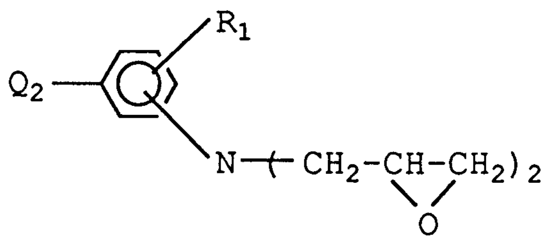 ··· (2)

wherein $Q_2$ represents the same group as $Q_1$ in the general formula (1), and $R_1$ represents hydrogen atom or methyl group, the epoxy resins (A-1) and (A-2) being present at a ratio of (A-1)/(A-2) = 100/0 to 50/50 by weight,

(B) at least one compound selected from the group consisting of dicyandiamide, 2,6-xylenylbiguanide, o-tolylbiguanide, diphenylguanidine, adipyl dihydrazide, azelayl dihydrazide, and isophthalic acid dihydrazide,

(C) a compound represented by the following general formula:

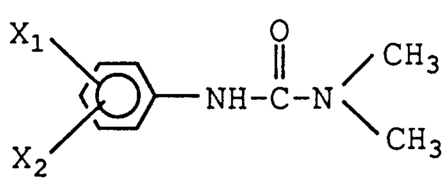 ··· (3)

wherein $X_1$ and $X_2$, which may be the same or different, each represents -H, -Cℓ, -Br, $-NO_2$, $-CH_3$, $-OCH_3$, $-OC_2H_5$, or

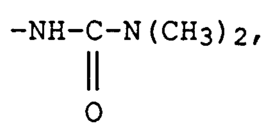

and

(D) a reaction product obtained by reacting an amide or amine represented by the general formula:

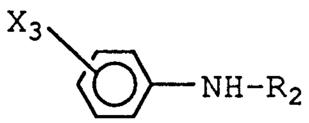

... (4)

wherein $X_3$ represents -H, -Cℓ, -Br or -OH, and $R_2$ represents -H, $-COCH_3$, or $-CO-CH_2-CO-CH_3$ with an epoxy compound represented by the general formula:

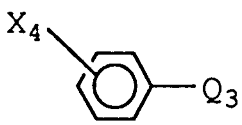

... (5)

or

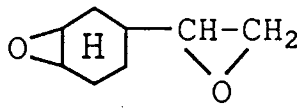

... (6)

wherein $X_4$ is the same as $X_3$ in the general formula (4) and $Q_3$ represent the same group as $Q_1$ in the general formula (1); containing

(II) about 60 volume %, based on the cmposite, of a unidirectionally oriented fiber reinforcement.

**22.** The composite of claim 21, wherein the amount of the compound (B) is 1 to 10 parts by weight per 100 parts by weight of the epoxy component (A).

**23.** The composite of claim 21, wherein the compound (B) is dicyandiamide.

**24.** The composite of claim 21, wherein the amount of the compound (C) is 1.5 to 15 parts by weight per 100 parts by weight of the epoxy component (A).

**25.** The composite of claim 21, wherein the compound (C) is 3-(3,4-dichlorophenyl)-1,1-dimethylurea.

**26.** The composite of claim 21, wherein the amount of the reaction product (D) is 5 to 100 parts by weight per 100 parts by weight of the total amount of the epoxy component (A), the compound (B), and the compound (C).

**27.** The composite of claim 21, wherein the reaction product (D) is a reaction product of p-hydroxyacetanilide or acetanilide with phenyl glycidyl ether.

**28.** The composite of claim 21, wherein the reaction product (D) is a reaction product of aniline with 4-vinyl cyclohexene dioxide.

**29.** The composite of claim 21, wherein the resin composition further contains up to 50% by weight of an epoxy resin other than the epoxy resins (A-1) and (A-2).

**30.** The composite of claim 21, wherein the fiber reinforcement comprises glass fibers, organic fibers or

metal fibers.

**31.** The composite of claim 21, wherein the fiber reinforcement comprises carbon fibers.